# EUROPEAN PATENT APPLICATION

(11) **EP 4 421 522 A1**
(43) Date of publication of application: **28.08.2024**
(21) Application number: 24158434.1
(22) Date of filing: 19.02.2024
(51) Int. Cl.: G01S 7/52, G01S 15/89

(54) **MULTI-APPLICATION ULTRASOUND MATRIX ARRAY SIGNAL PROCESSOR**

(30) Priority: 22.02.2023 US 202318172579
(71) Applicant: Siemens Medical Solutions USA, Inc., Malvern, PA 19355 (US)
(72) Inventor: PHELPS, Robert, Carnation, WA, 98014 (US); PETERSEN, David A., Fall City, WA, 98024 (US); SIMPSON, Terry E., Sammamish, WA, 98075 (US); MILLER, Steven, North Bend, WA, 98045 (US)
(74) Representative: Horn Kleimann Waitzhofer Schmid-Dreyer Patent- und Rechtsanwälte PartG mbB

(57) **Abstract**

Ultrasound imaging uses matrix arrays (100). A common design is used for an integrated circuit (140). The integrated circuit (140) is programmable so that the same design of integrated circuit (140) may be used with different types of arrays (100), such as matrix arrays (100) with different pitches. The design allows for one element (102) to connect with multiple, identical processing circuits (200) of the signal processor (140).

## Description

### BACKGROUND

The present embodiments relate to ultrasound imaging with a matrix (e.g., two-dimensional (2D)) transducer (XDCR) array. Signal processors developed for ultrasound matrix arrays include an identical, repetitive matrix of processing circuits to process signals from the acoustic elements of the array.

Two ways are used to connect the signal processor to the acoustic elements. The oldest is to provide a connection circuit, such as a flexible or printed circuit board (PCB), to route signals between signal processing circuits and the acoustic elements. The second is to mate the signal processing circuits to the acoustic matrix in a parallel plane and make connections for each element by aligning the matrix of acoustic elements to the matrix of signal processing circuits, thereby making the signal processing circuits an integral part of the acoustic stack. This second approach is the most effective, lowest cost, and simplifies what might include multiple 1000s of connections.

In this second approach, the pattern of signal processing circuits closely matches the dimensions of the acoustic matrix of elements and, therefore, restricts reuse of a given application specific integrated circuit (ASIC). The alignment from the acoustic transducer elements to electronic support circuits of the ASIC uses a circuit implemented with a sandwiched rigid or flexible printed circuit or a redistribution layer (RDL) laid on top of the ASIC. This alignment allows the patterns to be stretched or compressed to match each other. Processing circuits are used one-for-one to acoustic elements. In some cases, there are more acoustic elements than processing circuits with multiplexers between the processing circuits and the acoustic elements. Due to the close matching of ASIC with array, a different ASIC is expensively designed and manufactured for each different array type, such as arrays with different pitches of acoustic elements.

### SUMMARY

By way of introduction, the preferred embodiments described below include systems, methods, and/or computer readable storage media for ultrasound imaging with matrix arrays. A common design is used for an integrated circuit. The integrated circuit is programmable so that the same design of integrated circuit may be used with different types of arrays, such as matrix arrays with different pitches. The design allows for one element to connect with multiple processing circuits of the signal processor.

In a first aspect, a transducer system is provided for ultrasound imaging. An integrated circuit has transmit and/or receive circuits in cells. Each of the cells of the transmit and/or receive circuits is a first channel for transmit and/or receive beamformation, respectively. A routing layer is positioned between a two-dimensional array of transducer elements and the integrated circuit. The two-dimensional array, integrated circuit, and routing layer form a stack, and the routing layer electrically connects multiple of the cells to one of the transducer elements.

In one embodiment, the integrated circuit is programmable so that different numbers of cells are connectable to a same transducer element. For example, the integrated circuit is configured to at least partially beamform with the multiple cells electrically connected to the one transducer element operating together as a beamformer channel with a same delay. As another example, the integrated circuit is configured to at least partially beamform with the multiple cells electrically connected to the one transducer element operating separately to form different beams for signal from or two the one transducer element.

According to another embodiment, the integrated circuit is configurable to operate with different pitches and/or numbers of the transducer elements.

In yet another embodiment, the routing layer electrically connects each of the transducer elements to different sets of the cells. Each of the sets includes multiple of the cells exclusive to the set.

As another embodiment, the routing layer is a redistribution layer. In one embodiment, the routing layer is a flexible circuit material with traces configured to route from a first pitch of the transducer elements to a second pitch, different than the first pitch, of signal pads of the integrated circuit.

In an embodiment, the integrated circuit is an application specific integrated circuit as a semiconductor chip. The application specific integrated circuit is operable with different element pitches for different arrays. The application specific integrated circuit as stacked with the two-dimensional transducer array is configured for operation with a pitch of the transducer elements of the two-dimensional transducer array.

In a second aspect, a method is provided for manufacturing an ultrasound transducer with a multi-application signal processor. An array of elements having a first pitch is selected. The array is stacked with (1) an intermediate layer establishing a change in pitch from the first pitch to a second pitch of the multi-application signal processor and with (2) the multi-application signal processor. The multi-application signal processor is programmed to operate with the elements in the first pitch.

In one embodiment, selecting includes selecting from arrays with different pitches. The multi-application signal processor is configurable to operate with any of the different pitches.

In another embodiment, the multi-application signal processor is a plurality of signal processing nodes. Each of the nodes has identical transmit, receive, and signal processing circuits as the other nodes. Stacking includes stacking so that multiple of the signal processing nodes connect to each of the elements through the intermediate layer. For example, programming includes programming the multi-application signal processor to share drive current and dynamic range between nodes connected to a same one of the elements. As another example, programming includes programming the multi-application signal processor to form multiple beams from a same signal from a same one of the elements using the multiple of the signal processing nodes connected to that same one of the elements.

As one embodiment, the method further includes selecting a different array having a third pitch different than the first pitch, stacking the different array with (1) another intermediate layer establishing a change in pitch from the third pitch to the second pitch of another multi-application signal processor and with (2) the other multi-application signal processor, and programming the other multi-application signal processor to operate with the elements in the third pitch, the other multi-application signal processor and the multi-application signal processor having a same design. For example, a different number of nodes of the other multi-application signal processor connect with each element of the different array than of nodes of the multi-application signal processor connecting with each element of the array.

In a third aspect, a transducer system is provided for ultrasound imaging. An integrated circuit has transmit and/or receive circuits in cells, where each of the cells of the transmit and/or receive circuits is a first channel for transmit and/or receive beamformation, respectively. A routing layer is positioned between a two-dimensional array of transducer elements and the integrated circuit. The two-dimensional array, integrated circuit, and routing layer forming a stack, and the integrated circuit is programmable to operate with different numbers of cells connected to each of the elements.

In one embodiment, the integrated circuit is programmed to connect multiple of the cells to each of the elements. For example, the multiple cells connect to each element share drive current and dynamic range.

In another embodiment, the integrated circuit is an application specific integrated circuit, and the routing layer comprises a redistribution layer.

The present invention is defined by the following claims, and nothing in this section should be taken as a limitation on those claims. The various aspects described above may be used individually or in any possible combination. Other aspects and advantages are discussed below in conjunction with the preferred embodiments. These further aspects and advantages may be used independently of any of the aspects described above.

### BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.
Figure 1 is a block diagram of one embodiment of a transducer system connected with an ultrasound imager;
Figure 2 is a block diagram of one embodiment of a portion of the transducer system;
Figure 3 illustrates a cell arrangement of an integrated circuit with an overlaid acoustic element;
Figure 4 illustrates an example 2x overlay on the cell arrangement of Figure 3 of acoustic elements with different pitch than the cell arrangement;
Figure 5 illustrates example programmed connections of cells for the overlay of Figure 4;
Figure 6 illustrates an example 3x overlay of acoustic elements with different pitch than the cell arrangement;
Figure 7 illustrates example programmed connections of cells for the overlay of Figure 6;
Figure 8 is a flow chart diagram of one embodiment of a method for forming a matrix array.

### DETAILED DESCRIPTION OF THE DRAWINGS AND PRESENTLY PREFERRED EMBODIMENTS

A separate ASIC design is typically provided for each different type of transducer, such as for each matrix transducer with a separate pitch and/or number of elements. Effort is needed in software, ultrasound engineering, and clinical validation if different ASICs are used to support multiple applications. The development time and cost for building multiple acoustic matrix array solutions for multiple product applications is high. The overhead cost is high unless a given product has high yearly sales, which is typically not the case for ultrasound transducers.

A multi-application ultrasound matrix array signal processor is provided. The same signal processor (e.g., ASIC) may be used for various applications, such as transthoracic adult and pediatric, transesophageal adult and pediatric, volume, thin slice musculoskeletal (MSK) (e.g., fine pitch in one dimension and larger pitch in the other dimension), and/or peripheral vascular transducers. A single processor design includes an array of matrix array element support circuits (ESC). The same processor may be used for various applications corresponding to different array pitches and/or numbers of elements. Each signal processor created can support any one of multiple different types of transducers.

The distribution or routing layer (e.g., redistribution layer (RDL)) is designed for each application, enabling different processing and element areas. The routing layer can match support circuits to a plurality of element grids. For example, the support circuit grid will work for 1xN, NxM, or 1xM grids.

The routing layer is used to connect more than one signal processing node or support circuit to a single element, effectively sharing the characteristics such as drive current and dynamic range. The common signal processor supporting use with any one of multiple acoustic arrays does so with a gate array approach where a sea of identical element support circuits containing transmit, receive, and signal processing are combined in integers of one or more and attached to an array of elements. The element area is ideally but not limited to an N relationship with the element support circuit area. N can be 1 where the element area and element support circuit areas match. N can be more than 1 when the element area is larger, such as one-dimensional arrays or two-dimensional arrays with larger spacing between elements.

The ASIC design enables one or more ASICs to connect in parallel to one or more elements via routing. Transmit currents of support circuits sharing an acoustic element are summed. Receive and transmit signal-to-noise ratio may be increased by sharing of support circuits by one element. The azimuth and elevation grid of the support circuits of the signal processor can be area matched with the acoustic elements. Attached printed circuits or other routing layers can extend array matching. Having multiple support circuits connect with a given acoustic element allows the signal processor to support forming a plurality of beams from the same element signals.

Having a common signal processor solution can minimize cost and time for successive applications and spread the development cost over a greater number of products. The signal processor design enables sharing in the analog signaling domain between the acoustic stack elements and the processing circuits. The support infrastructure such as clocks and controls on the common signal processor may be used with various applications.

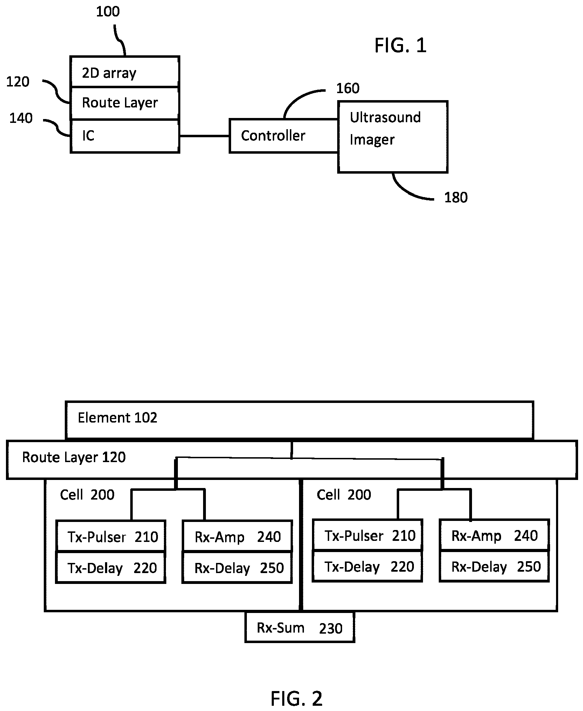

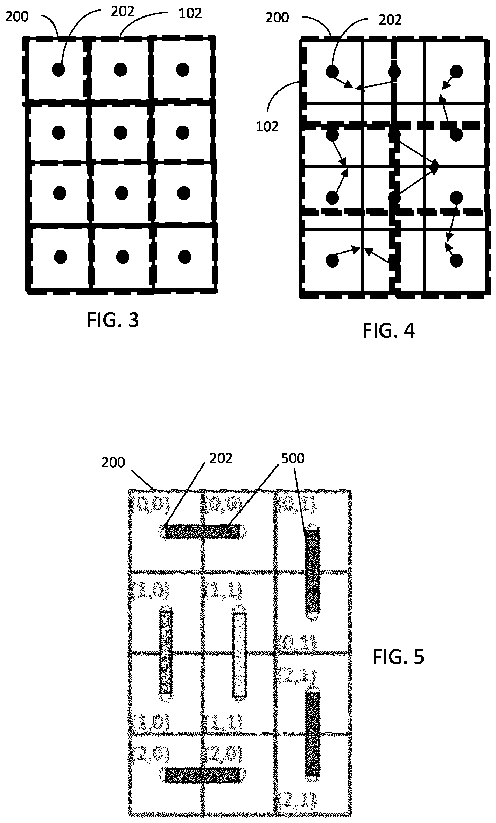

Figure 1 shows one embodiment of a transducer system for ultrasound imaging. The transducer system is formed from a matrix (2D) array 100. A signal processor (integrated circuit) 140 stacked with the array 100 has a common design that may be used for different applications. Figures 1 and 2 show the integrated circuit 140 being used in one application, but the design of the integrated circuit allows for use with transducers for different applications (compare Figures 3, 4, and 6). Rather than separate design, the integrated circuits 140 of the common design may be used in making transducers for any of the applications. For example, a box of chips of the integrated circuit 140 is provided. All of the chips have the same design. Chips from that box may be used to make any of various transducers. The integrated circuit 140 for any given transducer is programmed to operate with the transducer for which the chip was selected.

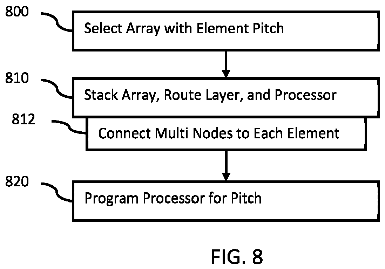

The transducer system is manufactured using the method of Figure 8 or another method. The transducer system operates with a controller 160 and/or ultrasound imager 180 for imaging a patient. The integrated circuit 140, as connected to the array 100 by the routing layer 120, is programmed to operate with the array 100 in the imaging.

The transducer system includes the 2D array 100, the routing layer 120, and the integrated circuit 140. The transducer system may physically and/or electrically connect with a controller 160 and an ultrasound imager 180. Additional, different, or fewer components may be provided. For example, in some embodiments, the routing layer 120 is integrated within and/or provided as part of the integrated circuit 140. In other embodiments, a beamformer, such as part of the ultrasound imager 180, is provided. The integrated circuit 140 provides partial beamformation (e.g., thousands of channels sub-array beamformed to 256, 192, 128, or 64 channels), and the beamformer of the imager 180 completes the beamformation (256, 192, 128, or 64 to beamformed samples representing spatial locations).

The 2D array 100 is a matrix array of transducer elements. The transducer elements are piezoelectric, CMUT, or PMUT elements. The elements are distributed in a fully sampled Cartesian grid as a 2D transducer array. Sparse sampling or other grid spacings of elements may be provided. Any number of elements may be provided, such as hundreds or thousands.

In some embodiments, the array of elements is positioned within a detachable transducer assembly. For example, the array 100 may be housed in a hand-held transducer housing. Alternatively, a catheter or endoscope configuration is used. The array may be designed for a specific application, such as selected pitch, area, shape, center frequency, and/or frequency range selected for imaging particular organs and/or through particular acoustic windows.

The 2D array 100 may have equal or unequal number of elements distributed in azimuth and elevation. For example, the 2D array 100 of the transducer elements has a greater number of the transducer elements along azimuth than a lesser number of the transducer elements along elevation. In one embodiment, the 2D array 100 is 72, 48, or 84 elements in azimuth and 80, 60, or 52 elements in elevation. Square, rectangular, circular, triangular, hexagonal, or other shaped arrays may be used.

The integrated circuit 140 is a processor in chip or wafer form, such as an application specific integrated circuit (ASIC), field-programmable gate array (FPGA), or another integrated circuit. ASIC will be used as the example herein, but other signal processors or integrated circuits may be used. The ASIC 140 provides for any signal processing, such as preamplification and/or digitization. The ASIC 140 may implement control functions, full beamformation, partial beamformation, and/or pulsers.

In some embodiments, the ASIC 140 is a partial beamformer for transmit and/or receive operation. For receive operation, the signals from separate elements 102 within a sub-array are combined to provide a sub-array signal as an output channel. Delay and sum, sum alone, and/or phase and sum partial beamformation may be provided for each sub-array 32, resulting in signals or data for each channel of the beamformer of the ultrasound imager 180. This partial beamformer for receive operation is a sub-array beamformer that beamforms separately for each sub-array to provide signals for each effective element (sub-array element) to the imager 180. The partially beamformed data is output to the system channels. From the perspective of the ultrasound imager 180, an entire sub-array represents a single array element because all receive signals for all elements within a sub-array are beamformed or relatively delayed and combined together before being forwarded to the imager 180. The system then beamforms the multitude of sub-array signals together.

For transmit, the ASIC 140 includes pulsers and drive controllers for each of various channels. Phasers and/or delays, amplifiers or other circuitry for amplification may be included with the pulsers and drive controllers for each channel to generate timed and apodized electrical waveforms for application to an acoustic element 102 for transmit beamformation.

The ASIC 140 is to operate with many elements of the array 100. The ASIC 140 includes support circuits (e.g., digitizers, preamplifiers, transmit channels, and/or receive channels) separated into many independent cells 200. Figure 2 shows an example of two cells adjacent each other in the ASIC 140. Tens, hundreds, or thousands of cells 200 or nodes with electrically isolated circuits are provided. The cells 200 may be distributed in any pattern, such as a Cartesian grid, with any pitch, such as a pitch that is the same or close (e.g., within 10%) of a pitch of the smallest elements 102 to be used for the array 100. For example, the pitch of the cells 200 is 142 pm by 159 pm. Other pitches with equal or unequal spacing in azimuth or elevation may be used.

The circuits of each cell 200 may be identical with the circuits of other cells 200. In the example of Figure 2, the circuit for a cell 200 includes pulsers 210 and delays or phasers 220 for transmit beamformation and delays 250 and amplifiers 240 for receive beamformation. A summer 230 as a component or as a mere connection between cells 200 may be provided for receive beamformation from the relatively delayed and apodised signals from the cells 200. Switches may be used to route received signals past the summer 230. Other shared circuits may be included in the ASIC 140, such as a controller. The cells 200 are each a channel for transmit and/or receive beamformation or partial beamformation. Other circuits repeated in each cell 200 may be included, such as analog-to-digital converters and preamplifiers.

Figure 3 shows an example of part of an ASIC 140 with cells 200 distributed in azimuth and elevation. A 3x4 cell arrangement is shown for simplification, as many more cells 200 in a larger array may be provided. The cells 200 are shown with nodes, pads, or electrical connections 202 for receiving signals from and providing signals to the acoustic element 102. In the example of Figure 3, one acoustic element 102 (dashed square) is shown for simplicity. Others would be provided. In this example, the elements 102 have a same or close pitch as the cells 200, so the node 202 of each cell exclusively connects to one element 102. The actual arrangement may be 72x80 cells 200 and elements 102 for an active area of 10.2 mm x 12.7 mm.

Figure 4 shows an example of the same ASIC 140 connected to an array 100 of elements 102 (dashed squares) where the elements 102 have a greater pitch, such as 213 pm x 212 pm for the elements 102 as compared to the 142 pm x 159 pm of the cells 200. The area of each element 102 in azimuth and elevation is larger than the area of each cell 200. In this example, the elements 102 are in a 2x3 arrangement over the 3x4 arrangement of cells 200. The actual arrangement may be 72x80 cells 200 and 48x60 elements 102 for an active area of 10.2 mm x 12.7 mm. As a result, there are twice as many nodes 202 as there are elements 102.

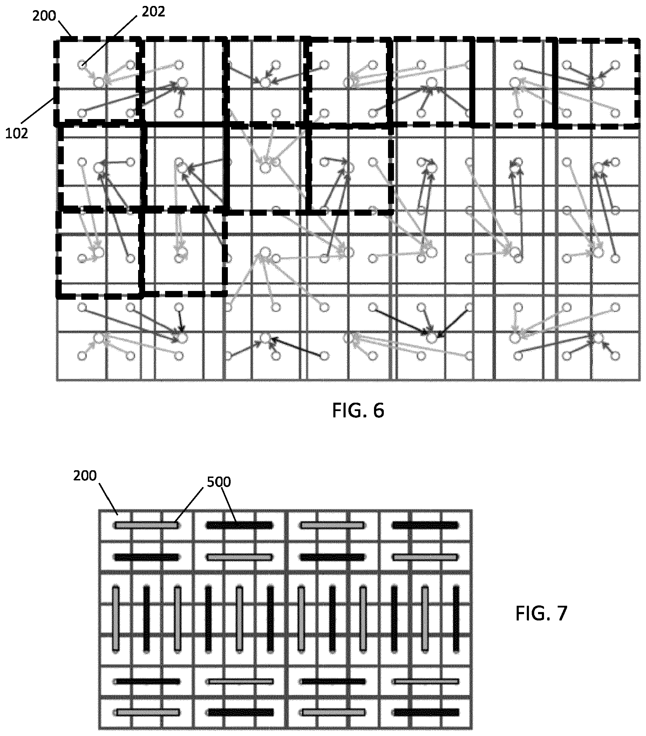

Figure 6 shows an example of a 7x12 array of cells 200 and corresponding nodes 202. In this example, the elements 102 (dashed squares shown over part of the ASIC 140) have a pitch of 243 pm x 248 pm, so there are three cells 200 for each element 102. The actual arrangement may be 72x91 cells 200 and 84x52 elements 102 for an active area of 10.2 mm x 12.9 mm.

Due to the mismatch in numbers, different elements 102 may have multiple cells 200 connected. Half, one-third, or other fraction of the cells 200 may be unused or not connected to elements 102. However, by connecting spare cells 200 to elements 102, the drive current and dynamic range may be shared between the cells 200 connected to the same element 102. Similarly, the same element signal may be processed separately, such as to form two different receive beams along different scan lines (i.e., apply different delay or phasing and apodization to the copies of the same signal).

Figure 2 shows an example where two cells 200 are connected to the same element 102. Figure 4 shows multiple of the cells 200 (two) connected to each of the elements 102. Figure 6 shows multiple cells 200 (three) connected to each of the elements 102. The arrows show pairs or triplets of nodes 202 connecting to the center or electrode of each of the elements 102. Four or more cells 200 may be connected to each element 102. Only one cell 200 may be connected to each element (e.g., see Figure 3). Different numbers of cells 200 may connect to different elements 102 in a same array 100.

The common ASIC 140 being capable of supporting multiple acoustic arrays 100 does so with a gate array approach where a sea of identical element support circuits (cells 200) containing transmit, receive, and signal processing are combined in integers of one or more and attached to an array 100 of elements 102. The element area is ideally but not limited to an N relationship with the element support circuit area. N can be 1 where the element area and element support circuit areas match (e.g., Figure 3). N can be more than 1 when the element area is larger such as one-dimensional arrays or two-dimensional arrays with larger spacing between elements (e.g., Figures 3 and 5).

Fractional matching is also possible where the overall ASIC 140 area is larger than the array 100 and the routing layer 120 compresses the connections. Fractional matching is also possible where the array 100 is larger and connected via an interposer, such as a rigid or flexible printed circuit as the routing layer 120.

The relationship does not need to hold for azimuth and/or elevation but rather the area. The spacing of the signal processors (cells 200) fixed in silicon or other semiconductor can have one aspect and size of azimuth and elevation while a second aspect and size preferred by one or more acoustic matrix patterns can be different. Generally, the area of these patterns is related not by the aspect but by the area and limited only by how the routing layer 120 can reach from one acoustic element 102 to an integer number of signal processing circuits (cells 200).

The same ASIC 140 is capable or configurable to operate with different arrays 100 having different pitches and/or numbers of transducer elements 102. The same design of ASIC 140 may be used in different types of arrays 100, arrays 100 for different applications, and/or arrays 100 with different frequency, bandwidth, and/or size (e.g., pitch and/or area) characteristics. While a given ASIC 140 itself may only ever connect to one array 100, the design of the ASIC 140 allows for ASICs 140 manufactured with the same die or design to be used with any of various arrays 100, increasing the number of ASICs 140 of the same design to be made while decreasing the design requirement to design a separate ASIC 140 for each type of array.

The ASIC 140 is programmed to connect multiple cells 200 to each element 102 so that the signal provided to the element 102 and/or received from the element 102 is handled appropriately (e.g., summed to form signal from one element or processed differently for forming multiple beams and/or sharing current for transmit waveform generation). The ASIC 140 is programmable to operate with different numbers of cells 200 connected to each of the elements 102. The routing layer 120 is used to connect more than one signal processing node 202 to a single element 102 effectively sharing the characteristics such as drive current and dynamic range. Furthermore, the element circuits (cells 200) are intended to benefit from multiple circuits being attached to a single element 102 because transmitter currents can be summed, and the returned element signal can be shared by multiple processors. The routing, switches and/or control are used so that the cells 200 shared with each element 102 operate together as common transmit and/or receive signals are to be used. The ASIC 140 is configured to at least partially beamform with the multiple cells 200 electrically connected to each transducer element 102 operating together as a beamformer channel with a same delay and/or apodization. Alternatively, the ASIC 140 is configured to at least partially beamform with the multiple cells 200 electrically connected to each transducer element 102 operating separately to form different beams for a signal from or to the one transducer element 102.

The ASIC 140 is a semiconductor chip. For a given array, a single ASIC 140 is used. In alternative embodiments, multiple ASICs 140 are tiled to connect to different parts of a common array 100.

The routing layer 120 is flexible circuit material (flex), deposited/etched conductors (e.g., traces and vias), redistribution layer (RDL), interposer with conductors, wires, printed circuit board, and/or another support structure with conductors for electrically connecting the nodes 202 to the elements 102. Vias, traces, wires, and/or other conductors and routing may be used.

The routing layer 120 is positioned between the two-dimensional array 100 of transducer elements 102 and the ASIC 140. As shown in Figure 1, the array 100, routing layer 120, and ASIC 140 are stacked and bonded. Asperity contact with epoxy or other bonding, lamination, flip-chip bonding, and/or solder may be used to physically connect the layers of the stack and/or to form conductive connections from the ASIC 140 to the array 100.

The ASIC 140 as stacked with the two-dimensional transducer array 100 is configured for operation with a pitch of the transducer elements 102 of the two-dimensional transducer array 100. The routing layer 120 routes the electrical connections. While the ASIC 140 may have a design that allows operation with various types of arrays 100, the routing layer 120 may be specifically designed for the array 100 and ASIC 140 combination. Since the routing layer 120 may be more cheaply and efficiently designed by type of array 100, the routing layer 120 may be different depending on type of array 100 while the ASIC 140 is designed to operate with any of multiple different arrays 100.

The routing layer 120 has conductors that adjust the pitch from the pitch of the elements 102 to the pitch of the cells 200. The cells 200 with a common connection to a same element 102 may be electrically connected together in the routing layer 120 (see Figure 2). Alternatively, the electrode of the element 102 is used to connect, and/or switches (e.g., multiplexer) in the ASIC 140 are used to connect. The routing conductors (e.g., traces and vias on a single layer or multiple layer flexible circuit) route from one pitch to another, different pitch. Where the cell pitch and element pitch are the same, the routing maintains the pitch. The signal pads or nodes 202 of the cells 200 of the ASIC 140 are electrically connected to the electrodes of the elements 102.

Where the area of the cells 200 is different than the area of the elements 102, the routing layer 120 electrically connects multiple of the cells 200 to one of the transducer elements 102. Each element 102 may have multiple cells 200 connected. For example, as shown in Figures 4 and 6, the routing layer 120 electrically connects two and three, respectively, cells 200 and corresponding nodes 202 to each element 102. Sets of cells 200 (e.g., two or more) are connected to each element 102. Each cell 200 is only connected with one element 102 (exclusive connection). The routing layer 120 (e.g., RDL) is used to connect more than one signal processing node 202 to a single element 102 (see Figure 2), effectively sharing the characteristics such as drive current and dynamic range.

Through routing in the routing layer 120 and/or programming (e.g., switched connections) in the ASIC 140, the ASIC 140 may be used with various arrays. For example, Figure 3 shows a one-to-one connection of cells 200 to elements 102. The ASIC 140 is programmed so that each cell 200 is one channel for beamformation. As another example, Figure 4 shows a two-to-one connection of cells 200 to elements 102. The ASIC 140 is programmed so that pairs of cells 200 act as a channel together or act to share the same signal. Figure 5 shows programming linking the nodes 202 for this arrangement of Figure 4. The links 500 via routing and/or programming have any of various patterns to pair cells 200 to elements 102. In yet another example, Figure 6 shows a three-to-one connection of cells 200 to elements 102. The ASIC 140 is programmed so that triplets of cells 200 act as a channel together or act to share the same signal. Figure 7 shows programming linking the nodes 202 for this arrangement of Figure 6. The links 500 via routing and/or programming have any of various patterns to connect three cells 200 to each element 102.

The array 100, route layer 120, and integrated circuit 140 are stacked in parallel planes. The route layer 100 and/or array 100 may have a curved or spherical surface. An intervening layer may be included where the intervening layer is curved for the array 100 and planar for the integrated circuit 140. The intervening layer includes z-axis connections.

The controller 160 is a processor, such as field-programmable gate array (FPGA), ASIC, general processor, or control processor. The controller 160 controls the ASIC 140. The controller 160 may control partial beamforming, such as configuring the delays or phasing and/or amplification to use and/or turning off partial beamforming where the signals from different elements 102 are combined without phasing or delay. The controller 160 is positioned in a connector of the transducer probe, such as the connector for releasable connection with the ultrasound imager 180. In other embodiments, the controller 160 is in the ultrasound imager 180, in the ASIC 140, and/or in the probe head with the transducer array 100.

Software, firmware, and/or hardware configure the controller 160 and/or ASIC 140 to program the ASIC 140 to operate with the cell-to-element configuration for a given array 100. Since a common ASIC 140 design is used, the programming configures the ASIC 140 for the particular array 100 with which the ASIC 140 is being used. Different ASICs 140 of the same design are programmed differently due to different numbers of cells 200 per element 102 being used for the transducer stack or system. Routing switches in the ASIC 140 may be controlled.

The ultrasound imager 180 is a medical diagnostic ultrasound imaging system. Transmit and receive beamformers connect through a transmit receive switching system to a connector for connection with a connector of an ultrasound probe. Where the ASIC 140 provides partial beamforming, the partially beamformed samples from the ASIC 140 are further beamformed by the beamformer of the ultrasound imager 180. The receive beamformer beamforms the signals from the sub-arrays and outputs the beamformed data to an image former, such as a detector and scan converter. A three-dimensional renderer may be provided for volume (i.e., 3D) or four-dimensional imaging. A display displays the image resulting from an acoustic scan of the patient by the transducer.

Figure 8 is a flow chart diagram of one embodiment of a method for manufacturing and using an ultrasound transducer with a multi-application signal processor. A common signal processor (e.g., ASIC) design is used. Signal processors built with the same design may be used in multiple different types of transducers. As result of being able to be used for different applications, the overall cost and time of design is spread over a greater number of transducers to be sold.

The method is implemented to form one of the systems of Figures 1-7 or another system. Additional, different, or fewer acts may be provided. For example, act 812 is not performed where the grid of the signal processor is the same as the grid of the array (e.g., same or similar pitch where similar is +/- 5%). The acts are performed in the order shown (top to bottom or numerical) or another order.

In act 800, an array of elements is selected. The array is manufactured or is to be manufactured for a given application. The application may define the size of the array, desired resolution, and/or frequency of operation, which in turn define the area and pitch of elements. The array of elements and corresponding pitch are selected.

Different arrays and pitches are available for selection. Where the array is to be manufactured, any pitch may be possible. The available pitches may be limited to integer factors of area of the cell area of the multi-application signal processor (e.g., ASIC). A group of available arrays with corresponding pitches is established by the distribution of cells, nodes or support circuits of the multi-application signal processor. For example, Figures 3, 4, and 6 shows three different arrays with three different corresponding pitches, all of which may be used with a same design of ASIC. In other embodiments, a greater number of available arrays and pitches is provided by fractional or other arrangements of area of elements to support circuit distribution.

Given the application (e.g., TTE, TEE, hand-held (exterior transducer), endoscope, catheter, and/or another transducer for adult or pediatric scanning, a desired element pitch is determined. The available pitches most closely matching the desired pitch is selected or the desired pitch is used. The routing layer may be designed to route for fractional or integer relationship between the grids.

The array is then manufactured. For example, a composite is formed by dicing a PZT slab and filling with epoxy. In other embodiments, the PZT is stacked in act 810 and bonded to the routing layer prior to dicing.

In act 810, the array or slab to form the array is stacked with an intermediate layer, establishing a change in pitch from the array pitch to a pitch of the multi-application signal processor, and stacked with the multi-application signal processor. The parts of the stack are aligned so the electrical connections from the signal processor to the elements are formed. The intermediate layer is designed to provide the electrical connections given the signal processor pads or grid and the element grid (i.e., given the pitches).

The intermediate layer and/or the signal processor may be designed so that multiple of the signal processing nodes connect to each of the elements through the intermediate layer in act 812. Each of the nodes of the multi-application signal processor has identical transmit, receive, and signal processing circuits. Multiple of these same circuits may be connected to one element or one to be formed element. A given circuit only connects to one element or one to be formed element.

The multi-application signal processor is the same regardless of the selection of the array. Since the multi-application signal processor, given design of the intermediate layer for signal routing, works with any of various arrays and pitches, the signal processor is of a common design. The selection of the signal processor for manufacture of a particular array may be selection from a box or collection of such processors without having to select between different processor specifically designed for different arrays or applications.

After stacking, the stack is bonded or otherwise connected. The array may be bonded with epoxy to the intermediate layer. The intermediate layer may be soldered (e.g., solder bumps or flip-chip bonding) to the signal processor. The array may be formed by dicing after stacking and bonding.

In act 820, the multi-application signal processor is programmed to operate with the elements in the pitch of the selected array. The multi-application signal processor is programmed to share drive current and dynamic range between nodes or circuits connected to a same one of the elements. The linking is programmed so that circuits connected to a same element operate together. For example, the circuits for the same element are used to process the same signal differently to form multiple beams. As another example, the circuits for the same element are used together to share processing or process the same so that the signals may be combined.

Acts 800-820 may be repeated. A different application is desired, so an array with a different pitch than for another transducer is selected in act 800. The stack is formed in act 810, possibly with a different number of circuits of the signal processor connected to each element than for a different array. The multi-application signal processor is a different instance of the same design, so that design of signal processor may be stacked with the intermediate layer and array (e.g., PZT to be used to form the array). The intermediate layer may have a different conductor arrangement or routing due to the difference in array pitches. A different number of nodes connect to each element for the different transducers due to the difference in pitch of the selected arrays. The multi-application signal processor is programmed to operate with the resulting circuit-to-element arrangement. This signal processor has a same design as one used for a different cell-to-element arrangement, but the programming and design allow for such common design.

In a further act, an ultrasound imager performs ultrasound imaging with the transducer as configured and programmed. The stack or transducer system is used in a transducer probe so that when connected to the imager, the imager can scan a patient for the application appropriate for the transducer. For transmit operation, a transmit beamformer, such as in the signal processor, provides signals separately to individual acoustic elements. For receive operation, signals from individual acoustic elements are combined, such as by partial beamforming or connection to a common conductor. The sub-array signals from the different contiguous sets are provided through group outputs to the beamformer channels of the imager. The signals are beamformed and image formed to generate one or more images representing an interior of the patient. The image is displayed for diagnostic use.

While the invention has been described above by reference to various embodiments, it should be understood that many changes and modifications can be made without departing from the scope of the invention. It is therefore intended that the foregoing detailed description be regarded as illustrative rather than limiting, and that it be understood that it is the following claims, including all equivalents, that are intended to define the spirit and scope of this invention.

## Claims

1. A transducer system for ultrasound imaging, the transducer system comprising:
a two-dimensional array (100) of transducer elements (102);
an integrated circuit (140) having transmit and/or receive circuits in cells (200), each of the cells (200) of the transmit and/or receive circuits comprising a first channel for transmit and/or receive beamformation, respectively; and
a routing layer (120) positioned between the two-dimensional array (100) of transducer elements (102) and the integrated circuit (140), wherein the two-dimensional array (100), integrated circuit (140), and routing layer (120) form a stack, and the routing layer (120) electrically connects multiple of the cells (200) to one of the transducer elements (102).

2. The transducer system of claim 1, wherein the integrated circuit (140) is programmable so that different numbers of cells (200) are connectable to a same transducer element (102) and wherein the integrated circuit (140) is configured to at least partially beamform with the multiple cells (200) electrically connected to the one transducer element (102) operating together as a beamformer channel with a same delay.

3. The transducer system of claim 1, wherein the integrated circuit (140) is programmable so that different numbers of cells (200) are connectable to a same transducer element (102) and wherein the integrated circuit (140) is configured to at least partially beamform with the multiple cells (200) electrically connected to the one transducer element (102) operating separately to form different beams for signal from or two the one transducer element (102).

4. The transducer system of one of claims 1 to 3, wherein the integrated circuit (140) is configurable to operate with different pitches and/or numbers of the transducer elements (102), and wherein the routing layer (120) electrically connects each of the transducer elements (102) to different sets of the cells (200), each of the sets including multiple of the cells (200) exclusive to the set, and wherein the routing layer (120) comprises a redistribution layer.

5. The transducer system of one of claims 1 to 4, wherein the integrated circuit (140) comprises an application specific integrated circuit (140) as a semiconductor chip, the application specific integrated circuit (140) operable with different element (102) pitches for different arrays (100), the application specific integrated circuit (140) as stacked with the two-dimensional transducer array (100) configured for operation with a pitch of the transducer elements (102) of the two-dimensional transducer array (100).

6. A method for manufacturing an ultrasound transducer with a multi-application signal processor (140), the method comprising:
selecting (800) an array (100) of elements (102) having a first pitch;
stacking (810) the array (100) with (1) an intermediate layer (120) establishing a change in pitch from the first pitch to a second pitch of the multi-application signal processor (140) and with (2) the multi-application signal processor (140); and
programming (820) the multi-application signal processor (140) to operate with the elements (102) in the first pitch.

7. The method of claim 6, wherein selecting (800) comprises selecting (800) from arrays (100) with different pitches, the multi-application signal processor (140) configurable to operate with any of the different pitches.

8. The method of claim 6 or 7, wherein the multi-application signal processor (140) comprises a plurality of signal processing nodes (200), each of the nodes (200) having identical transmit, receive, and signal processing circuits as the other nodes (200), and wherein stacking (810) comprises stacking (810) so that multiple of the signal processing nodes (200) connect (812) to each of the elements (102) through the intermediate layer (120).

9. The method of one of claims 6 to 8, wherein programming (820) comprises programming (820) the multi-application signal processor (140) to share drive current and dynamic range between nodes (200) connected to a same one of the elements (102) or wherein programming (820) comprises programming (820) the multi-application signal processor (140) to form multiple beams from a same signal from a same one of the elements (102) using the multiple of the signal processing nodes (200) connected to that same one of the elements (102).

10. The method of claim 6, further comprising selecting (800) a different array (100) having a third pitch different than the first pitch, stacking (810) the different array (100) with (1) another intermediate layer (120) establishing a change in pitch from the third pitch to the second pitch of another multi-application signal processor (140) and with (2) the other multi-application signal processor (140), and programming (820) the other multi-application signal processor (140) to operate with the elements (102) in the third pitch, the other multi-application signal processor (140) and the multi-application signal processor (140) having a same design.

11. The method of claim 10, wherein a different number of nodes (200) of the other multi-application signal processor (140) connect with each element (102) of the different array (100) than of nodes (200) of the multi-application signal processor (140) connecting with each element (102) of the array (100).

12. A transducer system for ultrasound imaging, the transducer system comprising:
a two-dimensional array (100) of transducer elements (102);
an integrated circuit (140) having transmit and/or receive circuits in cells (200), each of the cells (200) of the transmit and/or receive circuits comprising a first channel for transmit and/or receive beamformation, respectively; and
a routing layer (120) positioned between the two-dimensional array (100) of transducer elements (102) and the integrated circuit (140);
wherein the two-dimensional array (100), integrated circuit (140), and routing layer (120) forming a stack, and the integrated circuit (140) is programmable to operate with different numbers of cells (200) connected to each of the elements (102).

13. The transducer system of claim 12, wherein the integrated circuit (140) is programmed to connect multiple of the cells (200) to each of the elements (102).

14. The transducer system of claim 13, wherein the multiple cells (200) connected to each element (102) share drive current and dynamic range.

15. The transducer system of one of claims 12 to 14, wherein the integrated circuit (140) comprises an application specific integrated circuit (140) and the routing layer (120) comprises a redistribution layer.
